# EUROPEAN PATENT APPLICATION

(11) **EP 4 438 031 A1**
(43) Date of publication of application: **02.10.2024**
(21) Application number: 23164454.3
(22) Date of filing: 27.03.2023
(51) Int. Cl.: A61K 8/20, A61K 8/36, A61K 8/60, A61Q 5/00, A61Q 5/02, A61Q 5/12, A61Q 19/00, A61Q 19/10, C07H 1/00, C07H 15/04

(54) **A PROCESS FOR PREPARING A COMPOSITION COMPRISING A CARBOXYALKYLATED ALKYL- AND/OR ALKENYLGLYCOSIDE, AND THE COMPOSITION OBTAINABLE BY THIS PROCESS**

(71) Applicant: KAO CHEMICALS GmbH, 46446 Emmerich (DE)
(72) Inventor: PETERS, Sarah, D-46446 Emmerich (DE); MYRDEK, Thomas, D-46446 Emmerich (DE)
(74) Representative: Hoffmann Eitle

(57) **Abstract**

The present invention provides a process of preparation of a composition comprising at least a carboxyalkylated alkyl- and/or alkenylglycoside (component (a)), wherein the process comprises the steps: i) providing at least one alkyl and/or alkenylglycoside (component (b)) of formula (I) in a reactor; ii)adding an halocarboxylic acid, salt or ester thereof; iii) adding an alkaline agent; wherein step ii) comprises ii') initial feeding from 0.3 to 50% wt., preferably from 0.5 to 250, more preferably from 1 to 10% of the total amount of halocarboxylic acid, salt or ester thereof intended to be added in step (ii),and stirring until its dissolution; and ii")feeding the rest of the halocarboxylic acid, salt or ester thereof.

R¹-[G]ₚ Formula (I)

## Description

### FIELD OF THE INVENTION

The present invention relates to a process of preparation of a composition comprising one or more carboxyalkylated alkyl- and/or alkenylglycosides, to a composition obtainable by such process of preparation, to cosmetic compositions comprising thereof and to the use of said cosmetic composition in hair and/or skin cleansing applications.

### STATE OF THE ART

Skin and hair cleanser compositions are required to have not only good detergency and cleansing ability, but they also need to be mild and be well tolerated by the skin and/or hair and do not cause excessive defatting or dryness to the skin and hair, with reduced tackiness, homogeneous, to be of easy applicability and to exhibit good foaming properties, such as foam stability, foam quantity, foam quality and foam smoothness. In addition, skin and hair cleanser compositions are demanded by consumers to exhibit good viscosity as well as good rheological properties of stability, feel and flow. Also, there is a concern in the customer products market towards the sustainability of cosmetic products. Thus, there is also a requirement to formulate products with ingredients that are considered "green" or "natural" since they are derived from renewable and/or sustainable sources.

Another requirement for the cosmetic ingredients is the reduction of their environmental footprint from manufacturing to their use as cosmetic ingredients. The obtention of more concentrated liquid products, thus, having lower water content, is associated to a reduction in energy consumption, as less water is being transported. Also, water content reduction makes possible to lessen the necessity for preservatives, since the low water content of concentrated products usually makes them an inadequate media for microorganism growth.

The preparation and use of anionic surfactants in skin and hair cleanser formulations is well known by the persons skilled in the art.

EP1436306 describes an aqueous surfactant mixture comprised of an alkyl- and/or alkenyl oligoglycoside ether carboxylic acid. The patent application describes the preparation method by reaction of an alkyl- and/or alkenyl oligoglycoside and an omega-halocarboxylic acid or a salt or ester thereof.

EP1385612 describes the use of a surfactant composition containing a surfactant mixture having an alkyl-and/or alkenyl oligoglycoside and an alkyl-and/or alkenyl oligoglycoside ether carboxylic acid. This patent application describes aqueous compositions wherein the foam requirements and tackiness requirements are fulfilled.

WO2021108518 describes citrate-functionalized alkyl polyglucoside derivatives, prepared by reaction of an epichlorohydrin/citric acid ester with an alkyl polyglycoside. Patent describes the use of the functionalized alkyl polyglucosides as viscosity builders and antiirritants in personal care cleansing products.

From the state of the art set forth above, it can be seen that there is still a need for further compositions comprising a carboxyalkylated alkyl-and/or alkenylglycoside compounds for skin and/or hair cleansing.

### SUMMARY OF THE INVENTION

The present invention aims at addressing the above-described needs, and provides a process of preparation of a composition comprising at least one carboxyalkylated alkyl- and/or alkenylglycoside compound; a composition obtainable by such process of preparation; and its use in cosmetic applications for, among others, skin and/or hair cleansing.

The compositions are able to comply with the requirements imposed on them and provide good performance.

Relevant properties of the carboxyalkylated alkyl- and/or alkenylglycosides of the invention are the ease of handling for the user, residual salt content, water content and low colour gradation. In particular, the compositions of the invention exhibit good viscosity, good rheological properties of stability, feel and flow, good foaming properties, such as foam stability, foam quantity, foam quality and foam smoothness, they can also be obtained at higher dry content matter, thus reducing its water content.

The compositions of the invention also have good detergency and cleansing ability; they are mild, well-tolerated by the skin and/or hair, do not cause excessive defatting or dryness to the skin and hair, show reduced tackiness, are homogeneous, and are easily applicable.

The inventors have developed a process which can confer or enhance at least some of the advantageous properties found in the compositions of the invention.

Thus, the first aspect of the present invention is related to a process for the preparation of a composition comprising at least one carboxyalkylated alkyl- and/or alkenylglycoside.

In a second aspect of the present invention provides the composition obtainable by the process as defined in the first aspect of the invention.

Cosmetic compositions comprising the composition as defined in the first and/or second aspect(s) of the invention are also an aspect of the invention.

Another aspect of the present invention is the use of any of the compositions provided by the invention for the care of skin and/or hair, in particular for cleansing skin and/or hair. This aspect can also be formulated as a method of cleansing or conditioning skin and/or hair with any of the compositions of the invention, the method comprising applying the composition to the skin and/or hair.

It is also an aspect of the present invention a process of preparation of the cleansing composition.

### DETAILED DESCRIPTION OF THE INVENTION

Terms not specifically defined herein should be given the meanings that would be given to them by one of skill in the art in light of the disclosure and the context. As used in the specification, however, unless specified to the contrary, the following terms have the meaning indicated and the following conventions are adhered to.

Throughout the present specification and the accompanying clauses, the words "comprise" and variations such as "comprises", "comprising" are to be interpreted inclusively. That is, these words are intended to convey the possible inclusion of other elements or integers not specifically recited, where the context allows. The word "comprise" also includes the term "consists of".

For the purposes of the present invention, any ranges given include both the lower and the upper end-points of the range.

All percentages are weight percentages, unless otherwise indicated.

As cosmetic composition it is understood a composition suitable as an ingredient to be used in cosmetic and personal care applications, in particular for skin and hair care.

The main object of the present invention is a process of preparation of a composition comprising:
a) at least a carboxyalkylated alkyl-and/or alkenylglycoside compound.

The terms "alkylglycoside compound" and "alkenylglycoside compound" refer to a sugar moiety (i.e. a saccharide, which may be a mono or oligosaccharide) which is derivatized by bonding one of its hydroxyl groups to an alkyl or alkenyl group, forming an ether bond, and wherein one or more of the remaining hydroxyl groups of the sugar moiety is/are each bonded to a moiety which at least comprises an halocarboxylic acid group or a salt thereof.

The process of preparation of a composition comprising at least a component (a), being a carboxyalkylated alkyl- and/or alkenylglycoside, comprises, in a first step, the provision of at least one alkyl and/or alkenylglycoside of formula (I) (hereinafter also referred as component (b)) in a reactor

R1-[G]ₚ Formula (I).

### Alkyl and/ or alkenylglycoside (component (b)):

According to the present invention, the process of preparation of the carboxyalkylated alkyl and/or alkenylglycoside compound comprises providing at least one alkyl and/or alkenylglycoside of formula (I)

R1-[G]ₚ Formula (I)

wherein R1 represents an alkyl or alkenyl group having from 4 to 24 carbon atoms, preferably from 10 to 18 carbon atoms, more preferably from 10 to 16 carbon atoms, even more preferably from 12 to 16 carbon; G group is a sugar unit containing 5 or 6 carbon atoms, preferably is a glucose.

The bonding of the R1- moiety to the sugar unit G is via an ether bond between R1 and one hydroxyl group of the sugar unit G; the bonding of two G groups (-G-G-) when p>1 represents the formation of a glycosidic linkage, i.e. an ether bond between one hydroxyl group from one of the sugar units G and one hydroxyl group from the other sugar unit G (like in any disaccharide, which results in the loss of a hydrogen atom from one sugar unit G and a hydroxyl group from the other sugar unit G).

The alkyl or alkenylglycoside can be derived from aldoses or ketoses containing 5 or 6 carbon atoms, i.e. G is a sugar unit derived from aldoses or ketoses containing 5 or 6 carbon atoms.

Examples of aldoses containing 5 or 6 carbon atoms are ribose, arabinose, xylose, lyxose and glucose. Examples of ketoses containing 5 or 6 carbon atoms are ribulose, xylulose, fructose, psocose, sorbose and tagatose.

Preferably, the alkyl or alkenylglycoside is derived from glucose, i.e. G is glucose.

The index p in the formula indicates the degree of oligomerization (i.e. the number of glycoside units, i.e. mono- and oligoglycosides), and it is a number from 1 to 10. Whereas p in a given compound must always be an integer and, preferably, it may assume a value of 1 to 6, the p value for a certain alkyl-and/or alkenylglycoside is an analytically determined quantity obtained by gas chromatography, which is generally not an integer but has decimals. The determination of the degree of oligomerization can be obtained following routine and well-known protocols to those skilled in the art. The skilled in the art would also recognize that substantially the same degree of oligomerization will be obtained using one or another protocol. So, in the context of the invention "p" encompasses also the "average degree of oligomerization" which can be determined as provided below. In an embodiment, the at least one alkyl- and/or alkenylglycoside has an average degree of oligomerization p from 1.1 to 3.0.

In an embodiment of the present invention, R1 is a linear or branched alkyl having from 4 to 24 carbon atoms or a linear alkenyl group having from 4 to 24 carbon atoms and from 1 to 3 double bonds; preferably the alkyl or alkenyl has from 10 to 18 carbon atoms, more preferably from 10 to 16 carbon atoms, even more preferably from 12 to 16 carbon atoms.

As used herein, the term "alkyl" refers to a straight or branched hydrocarbon chain having the indicated number of carbon atoms, preferably from 4 to 24 carbon atoms, more preferably from 10 to 18 carbon atoms, more preferably from 10 to 16 carbon atoms, even more preferably from 12 to 16 carbon atoms.

As used herein, the term "alkenyl" refers to a linear hydrocarbon chain having the indicated number of carbon atoms, preferably from 4 to 24 carbon atoms, more preferably from 10 to 18 carbon atoms, more preferably from 10 to 16 carbon atoms, even more preferably from 12 to 16 carbon atoms, and from 1 to 3 double bonds (i.e. 1, 2 or 3 double bonds).

Illustrative non-limitative examples of R1 groups are capryl, octanyl, nonanyl, decanyl, dodecanyl, tetradecanyl, hexadecanyl, hexadec-9-en-1-yl, octadecanyl, 16-methylheptadecan-1-yl, octadec-9-en-1-yl, octadeca-9,12-dien-1-yl, octadec-6-en-1-yl, docosanyl, and docos-13-en-1-yl.

In a preferred embodiment of the present invention, R1 is selected from dodecanyl and tetradecanyl.

In an embodiment of the present invention, the alkyl- and/or alkenylglycoside of formula (I), is one wherein R1 is a linear alkyl chain having from 10 to 16 carbon atoms, G represents a glucose and p is a number from 1 to 3. In a preferred embodiment of the present invention, the alkyl- and/or alkenylglycoside of formula (I) is a caprylyl/caprylglycoside, a myristylglycoside, a laurylglycoside, a cocoglycoside, or a combination thereof. In a more preferred embodiment, the alkyl- and/or alkenylglycoside of formula (I) is a laurylglycoside. In another preferred embodiment of the invention, the alkyl- and/or alkenylglycoside of formula (I) is a D-Glucopyranose, oligomeric, C10-16-alkyl glycoside.

In another embodiment of the present invention, the alkyl and/or alkenylglycoside is from natural origin (i.e. is derived from plants or vegetables).

The obtention of the alkyl and/or alkenylglycoside of formula (I)is well-known in the state of the art. Articles by Biermann et al. in Starch/Stärke 45, 281 (1993), and J.Kahre et al. in SÖFW Journal No.8, 598 (1995) can be cited as representative of the extensive literature available on this component.

### Process to prepare the composition:

The process of preparation of the composition comprising the carboxyalkylated alkyl and/or alkenylglycoside compound (component (a)) according to the invention comprises the steps of:
i) providing at least one alkyl and/or alkenylglycoside (component (b)) of formula (I) in a reactor

   R¹-[G]ₚ Formula (I)

   wherein R1, G and p are as defined above,
ii) adding an halocarboxylic acid, ester or salt thereof into the reactor,
iii) adding an alkaline agent,
wherein step ii) comprises
ii') initial feeding from 0.3 to 50% wt., preferably from 0.5 to 25%, more preferably from 1 to 10% of the total amount of halocarboxylic acid, salt or ester thereof intended to be added in step (ii), and stirring until its dissolution; and, then,
ii")feeding of the rest of the halocarboxylic acid, salt or ester thereof.

In the context of the invention, the term "halocarboxylic acid" refers to a hydrocarbon chain, either saturated or unsaturated, having from 1 to 10 carbon atoms, including one or more carboxylic groups (-COOH), and wherein an halogen atom takes the place of a hydrogen atom in the hydrocarbon chain.

In an embodiment of the invention, the halocarboxylic acid is of formula (II)

A-(CH₂)ₘ-COOX Formula (II)

Wherein A represents an halogen, m is a number from 1 to 5, and X is selected from the group consisting of hydrogen, an alkali metal, ammonium and alkaline earth metal.

The "ester" or "salt" of the organic acid suitable in the context of the invention, can be obtained following routine synthetic approaches to those skilled in the art.

In another embodiment of the invention, the reaction between the alkyl and/or alkenylglycoside of formula (I) and a halocarboxylic acid, salt or ester thereof is a carboxyalkylation reaction.

In an embodiment of the invention, the additions of step ii") and step iii) take place simultaneously, particularly under constant speed.

The term "constant speed" is the speed that guarantees that the reagents (i.e., the halocarboxylic acid, salt or ester thereof, and the alkaline agent) are independently added at the significantly same amount during all reaction time, until completing the addition steps.

In another embodiment, the halocarboxylic acid, salt or ester thereof from step ii"), and the alkaline agent from step iii) are independently fed to the reaction at a constant speed. By "independently fed" it is understood that the halocarboxylic acid, salt or ester thereof and the alkaline agent are added in independent additions (i.e. separate inlets) in the reactor.

In another embodiment, the halocarboxylic acid, salt or ester thereof from step ii"), and the alkaline agent from step iii) are added into the reactor, at a constant speed during 4 to 6 hours, preferably from 4.5 to 5.5 hours.

In another embodiment, reaction temperature is from 40 to 90°C, preferably from 40 to 80°C, more preferably from 40 to 70°C.

In an embodiment of the invention, the process further comprises the step iv) wherein after finalization of steps ii') and step iii), the reaction mixture is kept in the reactor, with constant stirring, from 3 to 5 hours.

In another embodiment, temperature in step iv) is from 40 to 100°C, preferably from 50 to 95°C, more preferably from 60 to 95°C.

In an embodiment of the invention, the molar ratio between the halocarboxylic acid, salt or ester thereof and the alkyl and/or alkenylglycoside of formula (I) is from 0.8:1 to 3:1, preferably from 0.9:1 to 2:1, more preferably from 0.9:1 to 1.25:1, even more preferably from 0.9:1 to 1.2:1.

In an embodiment of the invention, the molar ratio between the alkaline agent and the halocarboxylic acid, salt or ester thereof is from 1:1.05 to 1:1.1, preferably from 1:1.01 to 1:1.05, more preferably from 1:1 to 1.1:1.01.

In an embodiment of the invention, the halocarboxylic acid, salt or ester thereof is a salt of a monochloroacetic acid, preferably is sodium monochloroacetate.

In another embodiment, the carboxyalkylated alkyl- and/or alkenylglycoside compound (component (a)) is a carboxymethylated alkyl- and/or alkenylglycoside compound.

In another embodiment, the salt of monochloroacetic acid is in solid form.

In an embodiment of the invention, the alkaline agent is an alkali metal hydroxide or an alkaline earth metal hydroxide such as sodium hydroxide, potassium hydroxide, magnesium hydroxide, lithium hydroxide, barium hydroxide. Preferably the alkaline agent is selected from the group consisting of sodium hydroxide, potassium hydroxide, and mixtures thereof.

In another embodiment, the alkaline agent is added in solid form.

In another embodiment of the invention, the conversion degree of the carboxyalkylated alkyl and/or alkenyl glycoside is from 30 to 80%, preferably from 35 to 70%, more preferably from 40 to 60%.

Conversion degree is calculated as the moles of carboxyalkylated alkyl and/or alkenyl glycoside divided per moles of initial alkyl and/or alkenyl glycoside.

The composition that results from the reaction comprises the carboxyalkylated alkyl and/or alkenylglycoside (component (a)), unreacted substances in the process for obtaining the carboxyalkylated alkyl and/or alkenyl glycoside, such as alkyl and/alkenyl glycoside of formula (I), esters, impurities or byproducts from the same reaction such as sodium glycolate, sodium diglycolate, haloacetic acids, as well as as inorganic salts such as sodium chloride, and water.

In an embodiment of the present invention, the alkyl and/or alkenylglycoside of formula (I) corresponds to the non-derivatived species from the obtention of the carboxyalkylated alkyl and/or alkenylglycoside compound, (component (a)).

In another embodiment, the alkyl and/or alkenylglycoside of formula (I) is different from the corresponding non-derivatived species from the obtention of the carboxyalkylated alkyl and/or alkenylglycoside compound (component (a)). In this embodiment, the alkyl and/or alkenylglycoside of formula (I) would be added in a separate step to the reaction medium (prior or after any of the above-identified steps forming part of the process of the invention).

In an embodiment of the invention, the process further comprises the addition of a preservative agent, such as sorbic acid, sodium benzoate, potassium sorbate; preferably sorbic acid.

In another embodiment of the invention, the process further comprises the addition of a pH-modifier, such as citric acid, lactic acid, acetic acid, hydrochloric acid, sulfuric acid, phosphoric acid; preferably citric acid.

In an embodiment of the invention, the process of preparation of a composition comprising a carboxymethylated alkyl and/or alkenyl glycoside compound (component (a)) comprises the steps:
i) Providing an alkyl and/or alkenyl glycoside of formula (I) in a reactor,
   ii') initial feeding from preferably from 0.5 to 25%, more preferably from 1 to 10% of the halocarboxylic acid, salt or ester thereof intended to be added in step (ii), and stirring until its dissolution, and, then,
   ii")feeding of the rest of the halocarboxylic acid, salt or ester thereof.
iii) Addition of an alkaline agent;
iv) Maintenance of the reaction mixture, with constant stirring, from 3 to 5 hours, in the reactor;
Wherein:
additions of step ii") and step iii) take place: simultaneously, particularly under constant speed; particularly, the halocarboxylic acid, salt or ester thereof from step ii") and the alkaline agent from step iii) are simultaneously, but separately, added to the reactor; more particularly,
the halocarboxylic acid, salt or ester thereof from step ii') and the alkaline agent from step iii) are independently fed to the reaction.

In an embodiment of the invention, the process of preparation of a composition comprising a carboxymethylated alkyl and/or alkenyl glycoside compound (component (a)) comprises the steps:
ii) Providing an alkyl and/or alkenyl glycoside of formula (I) in a reactor,
ii') initial feeding from preferably from 0.5 to 25%, more preferably from 1 to 10% of the halocarboxylic acid, salt or ester thereof, and stirring until its total dissolution;
ii") feeding of the rest of the halocarboxylic acid, salt or ester thereof;
v) Addition of an alkaline agent;
vi) Maintenance of the reaction mixture, with constant stirring, from 3 to 5 hours, at temperature from 85 to 95°C, in the reactor;
wherein:
additions of step ii") and step iii) take place: simultaneously, particularly under constant speed; particularly, the halocarboxylic acid, salt or ester thereof from step ii") and the alkaline agent from step iii) are simultaneously, but separately, added to the reactor; more particularly, the halocarboxylic acid, salt or ester thereof from step ii') and the alkaline agent from step iii) are independently fed to the reaction;
the reaction temperature is from 40 to 70°C;
the halocarboxylic acid, salt or ester thereof is sodium monochloroacetate;
the alkaline agent is sodium hydroxide;
the alkyl and/or alkenylglycoside of formula (I) is one wherein R1 is a linear alkyl chain having from 10 to 16 carbon atoms, G represents a glucose unit and p is a number from 1 to 3; the molar ratio between the sodium monochloroacetate and the alkyl- and/or alkenylglycoside of formula (I) is from 0.9:1 to 1.2:1, wherein the molar ratio between sodium hydroxide and sodium monochloroacetate is from 1:1 to 1.1:1.01.

### Composition comprising at least one carboxyalkylated alkyl and/or alkenylglycoside obtainable by the process of the invention:

In another aspect of the present invention, the invention provides a composition comprising at least one carboxyalkylated alkyl and/or alkenylglycoside (component (a))obtainable by the process as defined in any of the embodiments provided above.

For the purposes of the invention the expressions "obtainable", "obtained" and equivalent expressions are used interchangeably, and in any case, the expression "obtainable" encompasses the expression "obtained".

All the embodiments provided above, regarding the process of the invention, among others, the steps, reagents, conditions, ratios, and % wt, are also embodiments of the composition obtainable by the process of the invention.

Preferably, the composition obtainable by the process of the present invention comprises at least a carboxyalkylated alkyl- or alkenylglycoside (component (a)) in a percentage by weight in the range from 15 to 60% wt., based on the total dry weight of the composition, preferably from 20 to 55% wt., more preferably from 30 to 55% wt.

In another embodiment of the invention, the total amount of component(s) (b) in the composition obtainable by the process of the present invention is from 15 to 60 %wt., preferably from 20 to 55% wt., more preferably from 30 to 55 %wt., with respect to the total dry weight of the composition.

In a preferred embodiment, in the composition obtainable by the process of the invention the ratio by weight of components (a):(b) is from 0.6:1 to 3.5:1, preferably from 0.5:1 to 2.5:1, more preferably from 0.5:1 to 1.5:1. In this embodiment, the "weight of component (a)" refers to the total amount of the one or more components (a) included in the composition; and "weight of component (b)" refers to the total amount of the one or more components (b) included in the composition, both weights being expressed in the same units.

In another embodiment, the composition obtainable by the process of the invention comprises:
- a component (a) in a percentage by weight in the range from 15 to 60% wt., based on the total dry weight of the composition, preferably from 20 to 55% wt., more preferably from 30 to 55% wt.;
- a component (b) of formula (I) as defined in any of the above embodiments, in a percentage by weight in the range from 15 to 60% wt., based on the total dry weight of the composition, preferably from 20 to 55% wt., more preferably from 30 to 55% wt.

In a preferred embodiment of the invention, the composition obtainable by the process of the invention comprises:
- a component (a), which is a carboxymethylated alkyl and/or alkenyl glycoside as defined in any of the above embodiments, in a percentage by weight in the range from 15 to 60% wt., based on the total dry weight of the composition, preferably from 20 to 55% wt., more preferably from 30 to 55% wt.

In another preferred embodiment, the composition obtainable by the process of the invention comprises:
- a component (a), which is a carboxymethylated alkyl and/or alkenyl glycoside as defined in any of the above embodiments, in a percentage by weight in the range from 15 to 60% wt., based on the total dry weight of the composition, preferably from 20 to 55% wt., more preferably from 30 to 55% wt.; and
- a component (b) of formula (I) as defined in any of the above embodiments, in a percentage by weight in the range from 15 to 60% wt., based on the total dry weight of the composition, preferably from 20 to 55% wt., more preferably from 30 to 55% wt.

In an embodiment of the invention, the content of inorganic salts in the final product (i.e. the composition obtainable by the process of the invention) is from 6 to 25% wt., based on the total dry weight of the composition, preferably from 7 to 20, more preferably from 7 to 16 %wt., with respect to the total dry weight of the composition.

In an embodiment of the invention, the content of inorganic salts in the final product (i.e. the composition obtainable by the process of the invention) is lower than 12% wt, preferably lower than 11% wt., more preferably lower than 10% wt., based on the total dry weight of the composition.

In the context of the invention, the expression "total dry weight of the composition" means the weight of all the constituents of the composition excluding water and it is determined by oven-drying method according to ISO 638.

In an embodiment of the invention, the content of sodium glycolate as by-product from the reaction is from 3 to 10% wt., preferably from 4 to 9% wt., more preferably from 4 to 7% wt., based on the total dry weight of the composition; and the content of sodium diglycolate as by-product from the reaction is from 0.2 to 7% wt., preferably from 0.4 to 4% wt., more preferably from 0.4 to 2% wt., based on the total dry weight of the composition.

Particularly, sodium glycolate and diglycolate are determined by reversed phase high-performance liquid chromatography with ultraviolet detector (HPLC-UV) in gradient mode using external standard calibration.

Quantitation is done by using analytical standards of these two products in the acidic form.

In another embodiment, the content of monohaloacetic acid, particularly monochloroacetic acid is below 20 ppm, based on the total dry weight of the composition.

In another embodiment, the content of dihaloacetic acid, particularly dichloroacetic acid is below 20 ppm, based on the total dry weight of the composition.

Particularly, monochloroacetic and dichloroacetic acid are determined by Ion chromatography (IC) coupled with ultraviolet detector (UV). External standard calibration of analytical standards of both products is used for their quantitation.

In another embodiment of the invention, the conversion degree of the carboxyalkylated alkyl and/or alkenyl glycoside is from 30 to 80%, preferably from 35 to 70%, more preferably from 40 to 60%.

Conversion degree is calculated as the moles of carboxyalkylated alkyl and/or alkenyl glycoside divided per moles of initial alkyl and/or alkenyl glycoside.

Particularly, unreacted alkyl- and/or alkenylglycoside is determined by reversed phase high-performance liquid chromatography with Evaporative Light-Scattering Detector (HPLC-ELSD) in gradient mode. Samples are dissolved in a mixture of water and organic solvent. Free alkyl- and/or alkenylglycoside percentage is obtained by external standard quantitation by comparison of retention times of a standard of C12-14 alkyl glycoside, oligomeric.

Particularly, the content of carboxyalkylated alkyl- and/or alkenylglycoside is determined by the difference of dry residue and the sum of all other species. Of the obtained result, it is deducted the % of polyglycoside alkylcarboxylate.

In another embodiment of the invention, the pH of the composition obtainable by the process of the invention is from 5 to 14, preferably from 7 to 12.

In another embodiment, the colour of the composition obtainable by the process of the invention is below 200(APHA), preferably below 150 (APHA), more preferably below 100 (APHA).

Particularly, colour of the compositions is determined after the addition of 3% of citric acid to each composition, based on the total weight of the composition. The measurement is done at 20°C and 35% of dry content, 24 hours after preparation. Colour is determined following ISO6271.

In another embodiment, the viscosity of the composition obtainable by the process of the invention is from 10 to 1700 cP, preferably from 20 to 900 cP, more preferably from 50 to 800 cP, measured at 20°C, 24 hours after preparation, with a Brookfield viscometer model LV.

In another embodiment, the viscosity of the composition obtainable by the process of the invention is below 500 cP, preferably below 400 cP, more preferably below 200 cP, even more preferably below 100 cP.

### Cosmetic composition:

Another aspect of the present invention is a cosmetic composition comprising the composition obtainable by the process as defined in any of the aspects and embodiments provided above and one or more cosmetically acceptable excipients and/or carriers.

In one embodiment, the cosmetic composition comprises component (a) and (b), and water as excipient.

In an embodiment of the present invention, the amount of carboxyalkylated alkyl and/or alkenylglycoside compound, i.e. component (a) in the cosmetic composition of the invention is from 0.5 to 15% wt. based on the total weight of the cosmetic composition, preferably from 1.2 to 10% wt., even more preferably from 2 to 10% wt.

In another embodiment of the invention, the amount of alkyl and/or alkenylglycoside, i.e. component (b) in the cosmetic composition is from 0.5 to 30 %wt. based on the total weight of the cosmetic composition, preferably from 1.2 to 20% wt., more preferably from 2 to 20 %wt.

In an embodiment of the invention, the cosmetic composition comprises:
- between 0.5 and 15%, preferably between 1.2 and 10%, more preferably between 2 and 10% wt. of component (a)
- between 00.5 and 30%, preferably between 1.2 and 20%, more preferably between 2 and 20 %wt. of component (b)
each of the indicated amounts being expressed as percentage by weight of the mentioned component with respect to the total weight of the cosmetic composition.

The cosmetic composition of the present invention may further comprise an additional component (c), said component being an amphoteric surfactant.

### Amphoteric surfactant (c):

Examples of amphoteric surfactant are alkyl amine oxides, alkyl betaines, alkyl sulphobetaines (sultaines), amidoalkyl betaines, alkyl glycinates, alkyl carboxyglycinates, alkyl amphoacetates, alkyl amphopropionates, alklylamphoglycinates, alkyl amidopropyl betaines, alkyl amidopropyl- and hydroxysultaines. Particularly preferred amphoteric surfactants are betaines, alkyl amine oxides, alkylamphoglycinates and alkyl amphoacetates.

In a preferred embodiment of the present invention, component (c) comprises one or more ampholytes. Specific examples of ampholytes are amine oxides. Suitable amine oxides according to the present invention are amine oxides with a hydrocarbon chain containing between 8 and 18 carbon atoms. Examples of commercially available amine oxides are those with the commercial reference OXIDET^{®} DM-20 (INCI name Lauramine Oxide), OXIDET^{®} DMCLD (INCI name Cocamine Oxide)OXIDET^{®} DM-246 (INCI name Cocamine Oxide), OXIDET^{®} DM-4 (INCI name Myristamine Oxide), OXIDET^{®} L-75 (INCI name Cocamidopropylamine Oxide), all of them marketed by KAO Chemicals Europe.

In a most preferred embodiment of the present invention, component (c) comprises one or more betaines. Specific examples of betaines are alkyl betaines, alkyl sulphobetaines (sultaines), amidoalkyl betaines, alkyl glycinates, alkyl carboxyglycinates, alkyl amphoacetates, alkyl amphopropionates, alkylamphoglycinates, alkyl amidopropyl betaines and hydroxysultaines. Particularly preferred betaines are alkyl amidopropyl betaines, alkyl amidopropyl hydroxysultaines, alkyl hydroxysultaines and alkyl amphoacetates. In a preferred embodiment the betaines are alkyl amidopropyl betaines.

In the context of component (c), the term "alkyl" refers to a straight or branched hydrocarbon chain having no unsaturations and having from 8 to 18 carbon atoms.

Examples of alkyl amidopropyl betaines are cocamidopropyl betaine, behenamidopropyl betaine, capryl/capramidopropyl betaine, lauramidopropyl betaine, myristamidopropyl betaine, palmamidopropyl betaine, palmitamidopropyl betaine and mixtures thereof.

Examples of commercially available useful betaine surfactants according to the invention are BETADET^{®} HR, BETADET^{®} HR-50K, BETADET^{®} S-20, BETADET^{®} SHR and BETADET^{®} THC-2, all marketed by Kao Chemicals Europe.

In another embodiment of the invention, the amount of amphoteric surfactant, i.e. component (c) in the composition is from 0.5 to 20%wt., preferably from 1.2 to 18% wt., more preferably from 2 to 10 %wt. with respect to the total sum of the cosmetic composition.

In a preferred embodiment, the ratio by weight of the sum of component (a) and component(b) to component (c) is from 5:1: to 1::20, preferably from 5:1 to 1:15:XX, more preferably from 5:1 to 1:10.

In an embodiment of the invention, the cosmetic composition comprises:
- between 0.5 and 15%wt., preferably between 1.2 and 10%wt., more preferably between 2 and 10% wt. of component (a)
- between 0.5 and 30% wt., preferably between 1.2 and 20%wt., more preferably between 2 and 20 %wt. of component (b)
- between 0.5 and 20%, preferably between 1.2 and 18%wt., more preferably between 2 and 10% wt. of component (c)
each of the indicated amounts being expressed as percentage by weight of the mentioned component with respect to the total weight of the cosmetic composition.

The pH of the cosmetic composition of the present invention is comprised between 3.5 and 7.5, preferably between 4.0 and 7.0, more preferably between 4.5 and 6.5.

The cosmetic composition according to the present invention may also comprise oil components, silicone compounds, powders, nonionic surfactants, anionic surfactants, polymers, metal ion sequestering agents, UV protection factors, vitamins, antioxidants, antioxidant aids, perfume oils, germ inhibitors and the like as further auxiliaries and additives.

Examples of oils include liquid oils, solid oils, waxes, hydrocarbon oils and synthetic ester oils. Suitable oil components are, for example, Guerbet alcohols based on fatty alcohols containing 6 to 22 and preferably 8 to 10 carbon atoms, esters of linear C6-C22 fatty acids with linear C6-C22 fatty alcohols, esters of branched C6-C22 carboxylic acids with linear C6-C22 fatty alcohols such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isopropyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl cleats, erucyl behenate and erucyl erucate. Also suitable are esters of linear C6-C22 fatty acids with branched alcohols, more particularly 2-ethyl hexanol, esters of hydroxycarboxylic acids with linear or branched C6-C22 fatty alcohols, esters of linear and/or branched fatty acids with polyhydric alcohols for example propylene glycol, dimer diol or trimer triol) and/or Guerbet alcohols, triglycerides based on C6-C10 fatty acids, liquid mono-/di-/triglyceride mixtures based on C6-C18 fatty acids, esters of C6-C22 fatty alcohols and/or Guerbet alcohols with aromatic carboxylic acids, more particularly benzoic acid, esters of C6-C12 dicarboxylic acids with linear or branched alcohols containing 1 to 22 carbon atoms or polyols containing 2 to 10 carbon atoms and 2 to 6 hydroxyl groups, vegetable oils such as avocado oil, almond oil, hazelnut oil, babassu palm oil, borage oil, peanut oil, jojoba oil, canola oil, hemp oil, soybean oil, milk thistle oil, safflower oil, chufa oil, coconut oil, rapeseed oil, black cumin oil, wheat germ oil, sunflower oil, linseed oil, macadamia nut oil, corn oil, walnut oil, olive oil, branched primary alcohols, substituted cyclohexanes, linear and branched C6-C22 fatty alcohol carbonates, Guerbet carbonates, esters of benzoic acid with linear and/or branched C6-C22 alcohols, linear or branched, symmetrical or non-symmetrical dialkyl ethers containing 6 to 22 carbon atoms per alkyl group, ring opening products of epoxidized fatty acid esters with polyols, silicone oils and/or aliphatic or naphthenic hydrocarbons, for example dialkyl cyclohexanes.

Examples of waxes include natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes, microwaxes; chemically modified waxes (hard waxes) such as for example, montan ester waxes, sasol waxes, hydrogenated jojoba waxes and synthetic waxes such as, for example, polyalkylene waxes and polyethylene glycol waxes.

Examples of hydrocarbon oils include liquid paraffin, squalane, pristane, paraffin, ceresin, squalene, petrolatum, and microcrystalline wax.

Suitable silicone compounds are, for example, dimethyl polysiloxanes, methylphenyl polysiloxanes, cyclic silicones and amino-, fatty acid-, alcohol-, polyether-, epoxy-, fluorine-, glycoside- and/or alkyl-modified silicone compounds which may be both liquid and resin-like at room temperature. Preferred silicone compounds are hydrophobic silicone oils, which are silicone oils which are soluble in paraffinic oil at 25°C. Hydrophobic silicone oils to be used according to the present invention include both volatile and non-volatile silicone oils.

Specific examples include a cyclic methyl siloxane having the formula {(CH₃₎₂SiO}ₓ in which x is 3-6, or short chain linear methyl siloxanes having the formula ((CH₃)₂SiO{(CH₃)₂SiO}_{y}Si(CH₃)₃ in which y is 0-5.

Some suitable cyclic methyl siloxanes are hexamethylcyclotrisiloxanes (D3), a solid with a boiling point of 134°C and the formula {(Me₂)SiO}₃; octamethylcyclotetrasiloxane (D4) with a boiling point of 176°C, a viscosity of 2.3 mm²/s, and the formula {(Me₂)SiO}₄; decamethylcyclopentasiloxane (D5) (cyclomethicone) with a boiling point of 210°C, a viscosity of 3.87 mm²/s, and the formula {(Me₂)SiO}₅; and dodecamethylcyclohexasiloxane (DE) with a boiling point of 245°C, a viscosity of 6.62 mm²/s and the formula {(Me₂)SiO}₆.

Some suitable short linear methyl siloxane are hexamethyldisiloxane (MM) with a boiling point of 100°C, viscosity of 0-65 mm<2>/s, and formula Me₃SiOMe₃; octamethyltrisiloxane (MDM) with a boiling point of 152°C., viscosity of 1.04 mm²/s, and formula Me₃SiOMe₂SiOSiMe₃; decamethyltetrasiloxane (MD2M) with a boiling point of 194°C, viscosity of 1.53 mm²/s, and formula Me₃SiO(MeSiO)₂SiMe₃; dodecamethylpentasiloxane (MD3M) with a boiling point of 229°C, viscosity of 2.06 mm²/s, and formula Me₃SiO(Me₂SiO)₃SiMe₃; tetradecamethylhexasiloxane (MD4M) with a boiling point of 245°C, viscosity of 2.63 mm²/s, and formula Me₃SiO(Me₂SiO)₄SiMe₃; and hexadecamethylheptasiloxane (MD5M) with a boiling point of 270°C, viscosity of 3.24 mm²/s, and formula Me₃SiO(Me₂SiO)₅SiMe₃.

Furthermore, long chain linear siloxanes such as phenyltrimethicone, bis(phenylpropyl)dimethicone, dimethicone, and dimethiconol are also included.

Examples of powders include inorganic powders such as talc, kaolin, mica, sericite, muscovite, phlogopite, synthetic mica, lepidolite, biotite, vermiculite, bentonite, hectorite, laponite, magnesium carbonate, calcium carbonate, aluminum silicate, barium silicate, calcium silicate, magnesium silicate, strontium silicate, tungstate, magnesium, silica, zeolite, barium sulfate, calcined calcium sulfate (calcined gypsum), calcium phosphate, fluorine apatite, hydroxyapatite, ceramic powder, metallic soap (e.g., zinc myristate, calcium palimitate, and aluminum stearate), and boron nitride; organic powders such as polyamide resin powder (nylon powder), polyethylene powder, polymethylmethacrylate powder, polystyrene powder, styrene-acrylic acid copolymer resin powder, benzoguanamine resin powder, poly(tetrafluroethylene) powder, and cellulose powder; inorganic white pigments such as titanium dioxide and zinc oxide; inorganic red pigments such as iron oxide (red iron oxide) and iron titanate; inorganic brown pigments such as γ-iron oxide; inorganic yellow pigments such as yellow iron oxide and ocher; inorganic black pigments such as black iron oxide and lower order titanium oxide; inorganic purple pigments such as mango violet and cobalt violet; inorganic green pigments such as chrome oxide, chrome hydroxide, and cobalt titanate; inorganic blue pigments such as ultramarine and Prussian blue; pearl pigments such as titanium oxide coated mica, titanium oxide coated bismuth oxychloride, titanium oxide coated talc, colored titanium oxide coated mica, bismuth oxychloride, and fish scale flakes; metal powder pigments such as aluminum powder and copper powder; organic pigments such as zirconium, barium, or aluminum lake (e.g., organic pigments such as Red No.201, Red No.202, Red No.204, Red No.205, Red No.220, Red No.226, Red No.228, Red No.405, Orange No.203, Orange No.204, Yellow No.205, Yellow No.401, and Blue No.404,or Red No.3, Red No.104, Red No.106, Red No.227, Red No.230, Red No.401, Red No.505, Orange No.205, Yellow No.4, Yellow No.5, Yellow No.202, Yellow No.203, Green No.3, and Blue No.1); and natural colors such as chlorophyll and β-carotene.

Examples of lipophilic nonionic surfactants include sorbitan fatty acid esters (such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate); glycerol or polyglycerol fatty acid esters (such as glycerol mono-cotton seed oil fatty acid ester, glycerol monoerucate, glycerol sesquioleate, glycerol monostearate, glycerol-a, a'-oleate pyroglutamate, and glycerol monostearate malate); propylene glycol fatty acid esters (such as propylene glycol monostearate); hardened castor oil derivatives; and glycerol alkyl ethers.

Examples of hydrophilic nonionic surfactants include POE-sorbitan fatty acid esters (such as POE-sorbitan monooleate, POE-sorbitan monostearate, and POE-sorbitan tetraoleate); POE sorbitol fatty acid esters (such as POE-sorbitol monolaurate, POE-sorbitol monooleate, POE-sorbitol pentaoleate, and POE-sorbitol monostearate); POE-glycerol fatty acid esters (such as POE-monooleates, POE-glycerol monostearate, POE-glycerol monoisostearate, and POE-glycerol triisostearate); POE-fatty acid esters (such as POE-distearate, POE-monodioleate, and ethylene glycol distearate); POE-alkyl ethers (such as POE-lauryl ether, POE-oleyl ether, POE-stearyl ether, POE-behenyl ether, POE-2-octyldodecyl ether, and POE-cholestanol ether); Pluronic type surfactants (such as Pluronic); POE/POP-alkyl ethers (such as POE/POP cetyl ether, POE/POP 2-decyltetradecyl ether, POE/POP monobutyl ether, POE/POP hydrogenated lanolin, and POE/POP glycerol ether); tetra POE/tetra POP-ethylenediamine condensates (such as Tetronic); POE-castor oil or hardened castor oil derivatives (such as POE-castor oil, POE-hardened castor oil, POE-hardened castor oil monoisostearate, POE- hardened castor oil triisostearate, POE-hardened castor oil monopyroglutamate monoisostearate diester, and POE-hardened castor oil maleate); POE-beeswax lanolin derivatives (such as POE-sorbitol beeswax); alkanolamides (such as coconut oil fatty acid diethanolamide, lauric acid monoethanolamide, and fatty acid isopropanolamide); POE-propylene glycol fatty acid esters; POE-alkylamines; POE-fatty acid amides; sucrose fatty acid esters; alkylethoxydimethylamine oxides; and trioleyl phosphate.

Examples of anionic surfactant, are, for example, alkyl sulphates (such as C6-C22 alkyl sulphates, metal salts of said C6-C22 alkyl sulphates as well as the ammonium salts or the salts of the organic amines with alkyl or hydroxyalkyl substituents), alkyl ether sulfate surfactant type (such as C6-C22 alkyl ether sulphates containing 0.5 to 5, preferably 0.8 to 3, moles of ethylene oxide per mol of the C6-C22 alkyl ether sulphate, metal salts of said alkylC6-C22 ether sulphates as well as the ammonium salts of organic amines with alkyl or hydroxyalkyl substituents, examples are sodium lauryl ether sulphate, potassium lauryl ether sulphate, ammonium lauryl ether sulphate and mono-, di- and triethylethanolamine lauryl ether sulphates containing from 0.8 to 3 moles of ethylene oxide per mole of alkyl ether sulphate, or mixtures thereof). Anionic surfactants of the sulfosuccinate types are also possible examples, including the alkylC6-C22 sulfosuccinates and alkylC6-C22 ether sulfosuccinates, preferably the mono- and di-alkylC6-C22 sulfosuccinates and mono- and di-alkylC6-C22 ether sulfosuccinates containing from 0.5 to 10, preferably from 1 to 5 mol of ethylene oxide per mol of alkyl (eg, mono- or di-alkyl) C6-C22 ether sulfosuccinate, or mixtures thereof, it being possible to use the metal salts of said mono- and di-alkylC6-C22 sulfosuccinates and mono - and di-alkyl C6-C22 ether sulfosuccinates as well as ammonium salts or salts of organic amines with alkyl or hydroxyalkyl substituents.

Suitable cationic polymers are, for example, cationic cellulose derivatives such as, for example, the quaternized hydroxyethyl cellulose obtainable from Amerchol under the name of Polymer JR 400, cationic starch, copolymers of diallyl ammonium salts and acrylamides, quaternized vinyl pyrrolidone/vinyl imidazole polymers such as, for example, Luviquat (BASF), condensation products of polyglycols and amines, quaternized collagen polypeptides such as, for example, Lauryidimonium Hydroxypropyl Hydrolyzed Collagen, quaternized wheat polypeptides, polyethyleneimine, cationic silicone polymers such as, for example, Amodimethicone, copolymers of adipic acid and dimethylamino-hydroxypropyl diethylenetriamine (Cartaretine, Sandoz), copolymers of acrylic acid with dimethyl diallyl ammonium chloride, polyquaternium type polymers, polyaminopolyamides and crosslinked water-soluble polymers thereof, cationic chitin derivatives such as, for example, quaternized chitosan, optionally in micro-crystalline distribution, condensation products of dihaloalkyls, for example dibromobutane, with bis-dialkylamines, for example bis-dimethylamino-1,3-propane, cationic guar gum such as, for example, Jaguar CBS, Jaguar C-17, Jaguar C-16 of Celanese, quaternized ammonium salt polymers such as, for example, Mirapol A-15, Mirapol AD-1, Mirapol AZ-1 of Mirapol.

Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl, acetate copolymers, vinyl pyrrolidone/dimethylaminoethyl methacrylate/vinyl caprolactam terpolymers and optionally derivatized cellulose ethers and silicones.

Examples of UV protection factors include organic substances (light filters) which are liquid or crystalline at room temperature and which are capable of absorbing ultraviolet radiation and of releasing the energy absorbed in the form of longer-wave radiation, for example heat. UV-B filters can be oil-soluble or water-soluble. The following are examples of oil-soluble substances: 3-benzylidene camphor or 3-benzylidene norcamphor and derivatives thereof, for example 3-(4-methylbenzylidene)-camphor; 4-aminobenzoic acid derivatives, preferably 4-(dimethylamino)-benzoic acid-2-ethylhexyl ester, 4-(dimethylamino)-benzoic acid-2-octyl ester and 4-(dimethylamino)-benzoic acid Amylester; esters of cinnamic acid, preferably 4-methoxycinnamic acid-2-ethylhexyl ester, 4-methoxycinnamic acid propyl ester, 4-methoxycinnamic acid isoamyl ester, 2-cyano-3,3-phenylcinnamic acid-2-ethylhexyl ester (Octocrylene); esters of salicylic acid, preferably salicylic acid-2-ethylhexyl ester, salicylic acid-4-isopropylbenzyl ester, salicylic acid homomethyl ester; derivatives of benzophenone, preferably 2-hydroxy-4-methoxybenzo-phenone, 2-hydroxy-4-methoxy-4'-methylbenzophenone, 2,2'-dihydroxy-4-methoxybenzophenone; esters of benzalmalonic acid, preferably 4-methoxybenzalmalonic acid di-2-ethylhexyl ester; triazine derivatives such as, for example, 2,4,6-trianilino-(p-carbo-2'-ethyl-1'-hexyloxy)-1,3,5-triazine and Octyl Triazone; propane-1,3-diones such as, for example, 1-(4-tert.butylphenyl)-3-(4T-methoxyphenyl)-propane-1,3-dione; 2-phenylbenzimidazole-5-sulfonic acid and alkali metal, alkaline earth metal, ammonium, alkylammonium, alkanolammonium and glucammonium salts thereof; sulfonic acid derivatives of benzophenones, preferably 2-hydroxy-4-methoxybenzophenone-5-sulfonic acid and salts thereof; sulfonic acid derivatives of 3-benzylidene camphor such as, for example, 4-(2-oxo-3-bornylidenemethyl)-benzene sulfonic acid and 2-methyl-5-(2-oxo-3-bornylidene)-sulfonic acid and salts thereof.

Typical UV-A filters are, in particular, derivatives of benzoyl methane such as, for example 1-(4'-tert.butylphenyl)-3-(4'-methoxyphenyl)-propane-1,3-dione, 4-tert-butyl-4'-methoxydibenzoyl methane (Parsol 1789) or 1-phenyl-3-(4'-isopropylphenyl)-propane-1,3-dione.

The UV-A and UV-B filters may of course also be used in the form of mixtures. Besides the soluble substances mentioned, insoluble pigments, i.e. finely dispersed metal oxides or salts, may also be used for this purpose. Examples of suitable metal oxides are, in particular, zinc oxide and titanium dioxide and also oxides of iron, zirconium, silicon, manganese, aluminium and cerium and mixtures thereof. Silicates (talcum), barium sulfate and zinc stearate may be used as salts. The oxides and salts are used in the form of the pigments for skin-care and skin-protecting emulsions and decorative cosmetics. The particles should have an average diameter of less than 100 nm, preferably from 5 to 50 nm and more preferably from 15 to 30 nm. They may be spherical in shape although ellipsoidal particles or other non-spherical particles may also be used. The pigments may also be surface-treated, i.e. hydrophilicized or hydrophobicized. Typical examples are coated titanium dioxides such as, for example, Titandioxid T 805 (Degussa) or Eusolex T2000 (Merck). Suitable hydrophobic coating materials are, above all, silicones and especially trialkoxyoctyl silanes or simethicones. So-called micro- or nanopigments are preferably used in sun protection products. Micronized zinc oxide is preferably used.

Besides the two above-mentioned groups of primary protection factors, secondary protection factors of the antioxidant type may also be used. Secondary sun protection factors of the antioxidant type interrupt the photochemical reaction chain which is initiated when UV rays penetrate into the skin. Typical examples of suitable antioxidants are amino acids (for example glycine, histidine, tyrosine, tryptophane) and derivatives thereof, imidazoles (for example urocanic acid) and derivatives thereof, peptides, such as D,L-carnosine, D-carnosine, L-carnosine and derivatives thereof (for example anserine), carotinoids, carotenes (for example α-carotene, β-carotene, lycopene) and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, γ-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and hepta-thionine sulfoximine) in very small compatible dosages (also (metal) chelators (for example (α-hydroxyfatty acids, palmitic acid, phytic acid, lactoferrine), α-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example γ-linolenic acid, linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, (α-glycosyl rutin, ferulic acid, furfurylidene glucitol, carnosine, butyl hydroxytoluene, butyl hydroxyanisole, nordihydroguaiac resin acid, nordihydroguaiaretic acid, trihydroxy-butyrophenone, uric acid and derivatives thereof, mannose and derivatives thereof, Superoxid-Dismutase, zinc and derivatives thereof (for example ZnO, ZnSO4), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

Examples of metal ion sequestering agents include 1-hydroxyethane-1,1-diphosphonic acid, 1-hydroxyethane-1,1-diphosphonic acid 4Na salt, disodium edetate, trisodium edetate, tetrasorium edetate, sodium citrate, sodium polyphosphate, sodium metaphosphate, gluconic acid, phosphoric acid, citric acid, ascorbic acid, succinic acid, edetic acid, and trisodium hydroxyethyl ethylenediamine triacetate.

Examples of vitamins include vitamins A, B1, B2, B6, C, and E and the derivatives thereof; pantothenic acid and the derivatives thereof; and biotin.

Examples of antioxidants include tocopherols, dibutylhydroxytoluene, butylhydroxyanisole, and gallic acid esters. Examples of antioxidant aids include phosphoric acid, citric acid, ascorbic acid, maleic acid, malonic acid, succinic acid, lactic acid, fumaric acid, cephalin, hexametaphosphates, phytic acid, and ethylenediaminetetraacetic acid.

Suitable perfume oils are mixtures of natural and synthetic fragrances. Natural fragrances include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamon, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert-butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, filial and bourgeonal. Examples of suitable ketones are the ionones, α-isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable fragrance. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, labolanum oil and lavendin oil. The following are preferably used either individually or in the farm of mixtures: bergamot oil, dihydromyrcenol, lilial, lyral, citronellol, phenylethyl alcohol, α-nexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, β-damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

Typical examples of germ inhibitors are preservatives which act specifically against gram-positive bacteria such as, for example, 2,4,4'-trichloro-2'-hydroxydiphenyl ether, chlorhexidine (1,6-di-(4-chlorophenyl-biguanido)-hexane) or TCC (3,4,4'-trichlorocarbanilide). Numerous perfumes and essential oils also have antimicrobial properties. Typical examples are the active substances eugenol, menthol and thymol in clove, mint and thyme oil.

The cosmetic compositions of the invention can be routinary prepared, for example, by mixing the selected excipients with the composition as defined in any of the aspects and embodiments provided in the specification.

The cosmetic compositions can be used in a large number of types of product for the skin and/or the hair such as mousses, gels, masks for the face or the hair, conditioners, formulations for improving hairstyling, or for facilitating the combing or disentangling of the hair, for providing volume or sheen, rinsing formulations, compositions for dying or colouring the hair, hand and body lotions and oils, products for improving the moisturization of the skin, cleansing milks, make-up-removing compositions, creams or lotions for protecting against the sun and ultraviolet radiation, care and/or treatment milks and creams, anti-acne preparations, local analgesics, mascaras, products intended to be applied to the lips or other mucous membranes, sticks, deodorant and antiperspirant products, shaving lotions, bath oils, talcs and other compositions of the same type.

In another embodiment, the compositions of the invention are used for hair care and/or for skin care. In another embodiment, the compositions of the invention can be used in compositions for the cleansing of skin and/or hair, hair dyeing compositions, and compositions for conditioning and moisturizing of skin and/or hair.

Thus, a further aspect refers to the use of the cosmetic compositions of the invention for the care of skin and/or hair selected from the group consisting of cleansing of skin and/or hair, conditioning and moisturizing of skin and/or hair, and the dyeing of hair, preferably cleansing of skin and/or hair.

In a preferred embodiment, the cosmetic compositions of the invention are used for the cleansing of skin and/or hair.

A method of cleansing skin and/or hair is also part of the invention, wherein the method comprises the steps of wetting or dampening the skin and/or hair, applying the cosmetic composition according to the invention on the skin and/or hair, and rinsing the skin and/or hair with water.

A method of conditioning human hair and/or skin, wherein the method comprises the steps of applying the cosmetic composition according to the invention to wet hair and/or wet skin, and either rinse it off from the hair and/or skin with water, or alternatively be left on hair and/or skin, is also a part of the invention.

In particular, in the methods defined above a sufficient amount of the composition of the invention is applied. The term "sufficient amount" refers to the amount of cosmetic composition necessary to achieve the cleansing or conditions of skin and/or hair and can be easily determined by the skilled person. An example of a sufficient amount is from 0.2g to 20g, more preferably from 0.5g to 15g..

The following examples are given in order to provide a person skilled in the art with a sufficiently clear and complete explanation of the present invention, but should not be considered as limiting of the essential aspects of its subject, as set out in the preceding portions of this description.

### EXAMPLES:

The first part of the Examples section corresponds to the process of preparation of the compositions according to the invention.

The second part of the Examples presents physical-chemical characteristics of the prepared composition.

### Example 1

### Preparation of Composition A (comparative)

643,6 g (0,73 mol) of C12/14 alkyl polyglycoside were placed in a stirred tank and heated to 60°C. After the temperature was reached, 29,1 g (0,73 mols) of NaOH prills were added and the mixture was stirred for one hour. 84,7 g (0,73 mols) of sodium monochloracetate were added and the mixture was stirred at 65°C for a further 3h. A viscous, light yellow liquid was formed. pH value (10% in H2O) was 11.7.

### Preparation of Composition B (according to the invention)

632,2 g (0,71 mol) of C12/14 alkyl polyglycoside were placed in a stirred tank and heated to 60°C. After the temperature was reached, 0,8 g (0,007 mol) of sodium monochloracetate was added and stirred until total dissolution. After that 83,1 g (0,71 mol) of sodium monochloracetate (0,28 g/min) and 28,6 g (0,71 mol) NaOH Prills (0,10 g/min) were dosed continuously at the same time over a period of 5h. After this the reaction mixture was stirred at 65°C for 3h. A viscous light yellow liquid was formed. pH value (10% in H2O) was 11.7.

### Example 2

Viscosity of the compositions is determined at 20°C and 33% of dry content, 24 hours after preparation, with a Brookfield viscometer model LV. Viscosity is measured using a spindle number 3 at 12 rpm.

Table 1 summarizes viscosity results:

**Table 1:**

| | Composition A | Composition B |
|---|---|---|
| Viscosity at 20°C and 33% dry matter , cP | 514 | 388 |

It can be seen that although both compositions include the same % of dry matter, in the case of the composition of the invention, which is obtained following a process wherein the halocarboxylic acid is added in two separate steps the viscosity is significantly lower when compared to composition A, obtained by a process wherein the addition of the acid is performed in a single step.

## Claims

1. A process of preparation of a composition comprising at least a carboxyalkylated alkyl- and/or alkenylglycoside wherein the process comprises the steps:
i) providing at least one alkyl and/or alkenylglycoside (component (b)) of formula (I) in a reactor
R¹-[G]ₚ Formula (I)
Wherein R1 represents an alkyl or alkenyl group having from 4 to 24 carbon atoms, preferably from 10 to 18 carbon atoms, more preferably from 10 to 16 carbon atoms, even more preferably from 12 to 16 carbon atoms; G is a sugar unit having 5 or 6 carbon atoms, preferably represents glucose; p is a number from 1 to 10;
ii) adding an halocarboxylic acid, salt or ester thereof;
iii) adding an alkaline agent;
wherein step ii) comprises
ii') initial feeding from 0.3 to 50% wt., preferably from 0.5 to 25%, more preferably from 1 to 10% of the total amount of halocarboxylic acid, salt or ester thereof intended to be added in step (ii),and stirring until its dissolution; and
ii")feeding the rest of the halocarboxylic acid, salt or ester thereof.

2. The process of preparation according to claim 1, wherein step ii") and step iii) take place simultaneously.

3. The process of preparation according to any of the claims 1 to 2, wherein the halocarboxylic acid, salt or ester thereof from step (ii'') and the alkaline agent from step (iii) are fed to the reaction each one at a constant speed.

4. The process of preparation according to any of the claims 1 to 3, wherein the molar ratio between the halocarboxylic acid, salt or ester thereof and the alkyl- and/or alkenylglycoside is from 0.8:1 to 3:1, preferably from 0.9:1 to 2:1, more preferably from 0.9:1 to 1.25:1

5. The process of preparation according to any of the claims 1 to 4, wherein the halocarboxylic acid, salt or ester thereof from step ii"), and the alkaline agent from step iii) are added into the reactor, each one at a constant speed during 4 to 6 hours, preferably from 4.5 to 5.5 hours.

6. The process of preparation according to any of the claims 1 to 5, wherein the halocarboxylic acid, ester or salt thereof is of formula (II)
A-(CH₂)ₘ-COOX Formula (II)
Wherein A represents an halogen, m is a number from 1 to 5, and X is selected from the group consisting of hydrogen, an alkali metal, ammonium and alkaline earth metal.

7. A composition comprising at least a carboxyalkylated alkyl- and/or alkenylglycoside compound which is obtainable by the process according to any of the claims 1 to 6.

8. A cosmetic composition comprising the composition according to claim 7, together with one or more cosmetically acceptable excipients and/or carriers.

9. Use of a composition according to claim 7, or of the cosmetic composition according to claim 8, for the care of skin and/or hair, particularly for cleansing of skin and/or hair.

10. A method to cleanse skin and/or hair comprising the steps of wetting or dampening the skin and/or hair, applying a sufficient amount of the composition according to claim 7, or alternatively of the cosmetic composition according to claim 8, on the skin and/or hair, followed by rinsing.

11. A method to condition skin and/or hair comprising the steps of applying the composition according to claim 7, or alternatively the cosmetic composition of claim 8, on the wet skin and/or hair, followed by rinsing it off from the hair and/or skin with water, or alternatively be left on the hair and/or skin.
